(19) Europäisches Patentamt / European Patent Office / Office européen des brevets

(11) EP 1 716 103 B1

(12) **EUROPÄISCHE PATENTSCHRIFT**

(45) Veröffentlichungstag und Bekanntmachung des Hinweises auf die Patenterteilung:
**10.01.2018 Patentblatt 2018/02**

(21) Anmeldenummer: **05701201.5**

(22) Anmeldetag: **27.01.2005**

(51) Int Cl.:
***C07C 253/10*** (2006.01) ***C07C 255/07*** (2006.01)

(86) Internationale Anmeldenummer:
**PCT/EP2005/000772**

(87) Internationale Veröffentlichungsnummer:
**WO 2005/073170 (11.08.2005 Gazette 2005/32)**

(54) **KONTINUIERLICHES VERFAHREN ZUR HERSTELLUNG VON LINEAREN PENTENNITRILEN**

CONTINUOUS METHOD FOR THE PRODUCTION OF LINEAR PENTENE NITRILES

PROCEDE POUR PRODUIRE EN CONTINU DES PENTENES NITRILES LINEAIRES

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **29.01.2004 DE 102004004696**

(43) Veröffentlichungstag der Anmeldung:
**02.11.2006 Patentblatt 2006/44**

(73) Patentinhaber: **BASF SE**
**67056 Ludwigshafen am Rhein (DE)**

(72) Erfinder:
- **BARTSCH, Michael**
  **67433 Neustadt (DE)**
- **BAUMANN, Robert**
  **68159 Mannheim (DE)**
- **HADERLEIN, Gerd**
  **67269 Grünstadt (DE)**
- **SCHEIDEL, Jens**
  **69493 Hirschberg (DE)**
- **JUNGKAMP, Tim**
  **B-2950 Kapellen (BE)**
- **LUYKEN, Hermann**
  **67069 Ludwigshafen (DE)**

(56) Entgegenhaltungen:
**EP-A- 0 274 401 WO-A-99/07671**
**WO-A2-97/36855**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 716 103 B1

**Beschreibung**

Die vorliegende Erfindung betrifft ein Verfahren zur kontinuierlichen Hydrocyanierung von 1,3-Butadien in Gegenwart eines Nickel(0)-Katalysators.

Adipodinitril, ein wichtiges Intermediat in der Nylonproduktion, wird durch zweifache Hydrocyanierung von 1,3-Butadien hergestellt. Dabei wird in einer ersten Hydrocyanierung 1,3-Butadien mit Cyanwasserstoff in Gegenwart von Nickel(0), das mit Phosphorliganden stabilisiert ist, zu Pentennitril umgesetzt. Hierbei bildet sich eine Mischung aus linearem 3-Pentennitril und verzweigtem Pentennitril (2-Methyl-3-butennitril). In einem zweiten Verfahrensschritt wird das verzweigte Pentennitril im Allgemeinen zu linearem Pentennitril isomerisiert. Abschließend wird das 3-Pentennitril in Gegenwart einer Lewis-Säure zu Adipodinitril hydrocyaniert.

Die Nickel(0)-katalysierte Hydrocyanierung von 1,3-Butadien zu Pentennitrilen in Abwesenheit von Lewis-Säuren und die Isomerisierung von 2-Methyl-3-butennitril zu 3-Pentennitril mit Hilfe von Nickel(0), das durch Phosphorliganden stabilisiert ist, ist an sich bekannt (WO 97/36855). Auch die Nickel(0)-katalysierte Hydrocyanierung von 1,3-Butadien zu Pentennitrilen in Gegenwart von Lewis-Säuren ist bekannt. Hierbei kommt es allerdings bei Einsatz eines mit monodentaten Phosphitliganden stabilisierten Nickel(0)-Katalysators zu einer unselektiven Bildung von linearen und verzweigten Dinitrilen wie Adipodinitril und Methylglutardinitril (W. C. Seidel, C. A. Toleman; Annals of the New York Academy of Science, Volume 415, Catalytic Transition Metal Hydrides, Seiten 201 bis 221, 1983). Für die Ausübung eines technischen Verfahrens ist die Selektivität der einzelnen Teilschritte von großer wirtschaftlicher und ökologischer Bedeutung, da beispielsweise die Kosten der genutzten Einsatzstoffe im Allgemeinen 70 % der Herstellungskosten ausmachen. Dass die bekannten Verfahren trotz der unselektiven ersten Hydrocyanierung Gesamtselektivitäten von größer 85 % erreichen und somit technisch und ökonomisch ausgeübt werden können, liegt unter anderem daran, dass die erste Hydrocyanierung von 1,3-Butadien in Abwesenheit von Lewis-Säuren auf der Hydrocyanierungsstufe der Pentennitrile stoppt und das unerwünschte verzweigte Pentennitrilisomer in das gewünschte lineare Isomer umgewandelt werden kann.

Für die kontinuierliche Synthese von Pentennitrilen aus 1,3-Butadien und Cyanwasserstoff ist es von Vorteil, 1,3-Butadien zu Cyanwasserstoff im molaren Verhältnis von 1 : 1 zu verwenden, um eine Rückführung des 1,3-Butadiens nicht vornehmen zu müssen. Dabei wurde jedoch festgestellt, dass bei einer solchen Fahrweise die Bildung von unerwünschtem Methylglutardinitril für ein wirtschaftliches Verfahren zu groß ist.

Die Aufgabe der vorliegenden Erfindung ist es somit, ein einfaches, selektives, katalysatorschonendes und kontinuierliches Verfahren zur Hydrocyanierung von 1,3-Butadien bereit zu stellen, bei dem die Bildung von Methylglutardinitril auf ein akzeptables Maß zurückgedrängt werden kann.

Gelöst wird diese Aufgabe durch ein Verfahren zur kontinuierlichen Hydrocyanierung von 1,3-Butadien in Gegenwart mindestens eines Katalysators, dadurch gekennzeichnet, dass man als Katalysatoren Nickel(0)-Katalysatoren, die mit phosphorhaltigen Chelatliganden stabilisiert sind, 1,3-Butadien und Cyanwasserstoff in einem molaren Verhältnis von 1,6 bis 1,1 zu 1 verwendet und dass die kontinuierliche Hydrocyanierung zusätzlich in Gegenwart mindestens einer Lewis-Säure durchgeführt wird. Erfindungsgemäß wurde gefunden, dass bei Verwendung von Nickel(0)-Katalysatoren mit den weiter unten beschriebenen Liganden ein Überschuss an 1,3-Butadien die Bildung von Methylglutardinitril zurückdrängt. Diese Feststellung steht dabei im Gegensatz zu der Lehre des Vergleichsbeispiels 1 der WO 98/27054, wo der Einsatz von einem größeren Überschuss an 1,3-Butadien zu einer schlechteren 1,3-Butadien-Selektivität führt.

Als Katalysator in dem erfindungsgemäßen Verfahren wird vorzugsweise ein homogen gelöster Katalysator verwendet. Besonders bevorzugt werden homogen gelöste Nickel(0)-Katalysatoren verwendet. Die besonders bevorzugten Nickel(0)-Katalysatoren sind mit phosphorhaltigen Chelatliganden stabilisiert.

Die Chelatliganden sind vorzugsweise besonders ausgewählt sind aus der Gruppe, bestehend aus bidentaten Phosphiten, Phosphinen, Phosphoniten, Phosphiniten und Phosphinitphosphiten.

Besonders bevorzugt Chelatliganden weisen die allgemeine Formel (I) auf

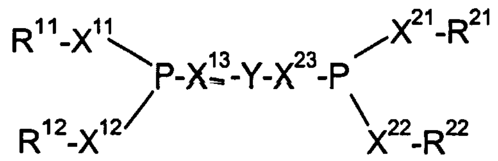

(I)

auf, worin bedeuten

$X^{11}$, $X^{12}$, $X^{13}$, $X^{21}$, $X^{22}$, $X^{23}$ unabhängig voneinander Sauerstoff oder Einzelbindung

$R^{11}$, $R^{12}$ unabhängig voneinander gleiche oder unterschiedliche, einzelne oder verbrückte orga-

nische Reste

| | |
|---|---|
| $R^{21}$, $R^{22}$ | unabhängig voneinander gleiche oder unterschiedliche, einzelne oder verbrückte organische Reste, |
| Y | Brückengruppe |

Unter Verbindung I wird im Sinne der vorliegenden Erfindung eine einzelne Verbindung oder ein Gemisch verschiedener Verbindungen der vorgenannten Formel verstanden.

In einer bevorzugten Ausführungsform können $X^{11}$, $X^{12}$, $X^{13}$, $X^{21}$, $X^{22}$, $X^{23}$ Sauerstoff darstellen. In einem solchen Fall ist die Brückengruppe Y mit Phosphit-Gruppen verknüpft.

In einer anderen bevorzugten Ausführungsform können $X^{11}$ und $X^{12}$ Sauerstoff und $X^{13}$ eine Einzelbindung oder $X^{11}$ und $X^{13}$ Sauerstoff und $X^{12}$ eine Einzelbindung darstellen, so dass das mit $X^{11}$, $X^{12}$ und $X^{13}$ umgebene Phosphoratom Zentralatom eines Phosphonits ist. In einem solchen Fall können $X^{21}$, $X^{22}$ und $X^{23}$ Sauerstoff oder $X^{21}$ und $X^{22}$ Sauerstoff und $X^{23}$ eine Einzelbindung oder $X^{21}$ und $X^{23}$ Sauerstoff und $X^{22}$ eine Einzelbiridung oder $X^{23}$ Sauerstoff und $X^{21}$ und $X^{22}$ eine Einzelbindung oder $X^{21}$ Sauerstoff und $X^{22}$ und $X^{23}$ eine Einzelbindung oder $X^{21}$, $X^{22}$ und $X^{23}$ eine Einzelbindung darstellen, so dass das mit $X^{21}$, $X^{22}$ und $X^{23}$ umgebene Phosphoratom Zentralatom eines Phosphits, Phosphonits, Phosphinits oder Phosphins, vorzugsweise eines Phosphonits, sein kann.

In einer anderen bevorzugten Ausführungsform können $X^{13}$ Sauerstoff und $X^{11}$ und $X^{12}$ eine Einzelbindung oder $X^{11}$ Sauerstoff und $X^{12}$ und $X^{13}$ eine Einzelbindung darstellen, so dass das mit $X^{11}$, $X^{12}$ und $X^{13}$ umgebene Phosphoratom Zentralatom eines Phosphonits ist. In einem solchen Fall können $X^{21}$, $X^{22}$ und $X^{23}$ Sauerstoff oder $X^{23}$ Sauerstoff und $X^{21}$ und $X^{22}$ eine Einzelbindung oder $X^{21}$ Sauerstoff und $X^{22}$ und $X^{23}$ eine Einzelbindung oder $X^{21}$, $X^{22}$ und $X^{23}$ eine Einzelbindung darstellen, so dass das mit $X^{21}$, $X^{22}$ und $X^{23}$ umgebene Phosphoratom Zentralatom eines Phosphits, Phosphinits oder Phosphins, vorzugsweise eines Phosphinits, sein kann.

In einer anderen bevorzugten Ausführungsform können $X^{11}$, $X^{12}$ und $X^{13}$ eine Einzelbindung darstellen, so dass das mit $X^{11}$, $X^{12}$ und $X^{13}$ umgebene Phosphoratom Zentralatom eines Phosphins ist. In einem solchen Fall können $X^{21}$, $X^{22}$ und $X^{23}$ Sauerstoff oder $X^{21}$, $X^{22}$ und $X^{23}$ eine Einzelbindung darstellen, so dass das mit $X^{21}$, $X^{22}$ und $X^{23}$ umgebene Phosphoratom Zentralatom eines Phosphits oder Phosphins, vorzugsweise eines Phosphins, sein kann.

Als Brückengruppe Y kommen vorzugsweise substituierte, beispielsweise mit $C_1$-$C_4$-Alkyl, Halogen, wie Fluor, Chlor, Brom, halogeniertem Alkyl, wie Trifluormethyl, Aryl, wie Phenyl, oder unsubstituierte Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste $R^{11}$ und $R^{12}$ können unabhängig voneinander gleiche oder unterschiedliche organische Reste darstellen. Vorteilhaft kommen als Reste $R^{11}$ und $R^{12}$ Arylreste, vorzugsweise solche mit 6 bis 10 Kohlenstoffatomen, in Betracht, die unsubstituiert oder einfach oder mehrfach substituiert sein können, insbesondere durch $C_1$-$C_4$-Alkyl, Halogen, wie Fluor, Chlor, Brom, halogeniertem Alkyl, wie Trifluormethyl, Aryl, wie Phenyl, oder unsubstituierte Arylgruppen.

Die Reste $R^{21}$ und $R^{22}$ können unabhängig voneinander gleiche oder unterscheidliche organische Reste darstellen. Vorteilhaft kommen als Reste $R^{21}$ und $R^{22}$ Arylreste, vorzugsweise solche mit 6 bis 10 Kohlenstoffatomen, in Betracht, die unsubstituiert oder einfach oder mehrfach substituiert sein können, insbesondere durch $C_1$-$C_4$-Alkyl, Halogen, wie Fluor, Chlor, Brom, halogeniertem Alkyl, wie Trifluormethyl, Aryl, wie Phenyl, oder unsubstituierte Arylgruppen.

Die Reste $R^{11}$ und $R^{12}$ können einzeln oder verbrückt sein. Auch die Reste $R^{21}$ und $R^{22}$ können einzeln oder verbrückt sein. Die Reste $R^{11}$, $R^{12}$, $R^{21}$ und $R^{22}$ können alle einzeln, zwei verbrückt und zwei einzeln oder alle vier verbrückt sein in der beschriebenen Art.

In einer besonders bevorzugten Ausführungsform kommen die in US 5,723,641 genannten Verbindungen der Formel I, II, III, IV und V in Betracht. In einer besonders bevorzugten Ausführungsform kommen die in US 5,512,696 genannten Verbindungen der Formel I, II, III IV, V, VI und VII, insbesondere die dort in den Beispielen 1 bis 31 eingesetzten Verbindungen, in Betracht. In einer besonders bevorzugten Ausführungsform kommen die in US 5,821,378 genannten Verbindungen der Formel I, II, III, IV, V, VI, VII, VIII, IX, X, XI, XII, XIII, XIV und XV, insbesondere die dort in den Beispielen 1 bis 73 eingesetzten Verbindungen, in Betracht.

In einer besonders bevorzugten Ausführungsform kommen die in US 5,512,695 genannten Verbindungen der Formel I, II, III, IV, V und VI, insbesondere die dort in den Beispielen 1 bis 6 eingesetzten Verbindungen, in Betracht. In einer besonders bevorzugten Ausführungsform kommen die in US 5,981,772 genannten Verbindungen der Formel I, II, III, IV, V, VI, VII, VIII, IX, X, XI, XII, XIII und XIV, insbesondere die dort in den Beispielen 1 bis 66 eingesetzten Verbindungen, in Betracht.

In einer besonders bevorzugten Ausführungsform kommen die in US 6,127,567 genannten Verbindungen und dort in den Beispielen 1 bis 29 eingesetzten Verbindungen in Betracht. In einer besonders bevorzugten Ausführungsform kommen die in US 6,020,516 genannten Verbindungen der Formel I, II, III, IV, V, VI, VII, VIII, IX und X, insbesondere die dort in den Beispielen 1 bis 33 eingesetzten Verbindungen, in Betracht. In einer besonders bevorzugten Ausführungsform kommen die in US 5,959,135 genannten Verbindungen und dort in den Beispielen 1 bis 13 eingesetzten Verbindungen in Betracht.

In einer besonders bevorzugten Ausführungsform kommen die in US 5,847,191 genannten Verbindungen der Formel I, II und III in Betracht. In einer besonders bevorzugten Ausführungsform kommen die in US 5,523,453 genannten Verbindungen, insbesondere die dort in Formel 1, 2, 3, 4, 5, 6, 7, 8, 9, 10, 11, 12, 13, 14, 15, 16, 17, 18, 19, 20 und 21 dargestellten Verbindungen, in Betracht. In einer besonders bevorzugten Ausführungsform kommen die in WO 01/14392 genannten Verbindungen, vorzugsweise die dort in Formel V, VI, VII, VIII, IX, X, XI, XII, XIII, XIV, XV, XVI, XVII, XXI, XXII, XXIII dargestellten Verbindungen, in Betracht.

In einer besonders bevorzugten Ausführungsform kommen die in WO 98/27054 genannten Verbindungen in Betracht. In einer besonders bevorzugten Ausführungsform kommen die in WO 99/13983 genannten Verbindungen in Betracht. In einer besonders bevorzugten Ausführungsform kommen die in WO 99/64155 genannten Verbindungen in Betracht.

In einer besonders bevorzugten Ausführungsform kommen die in der deutschen Patentanmeldung DE 100 380 37 genannten Verbindungen in Betracht. In einer besonders bevorzugten Ausführungsform kommen die in der deutschen Patentanmeldung DE 100 460 25 genannten Verbindungen in Betracht. In einer besonders bevorzugten Ausführungsform kommen die in der deutschen Patentanmeldung DE 101 502 85 genannten Verbindungen in Betracht.

In einer besonders bevorzugten Ausführungsform kommen die in der deutschen Patentanmeldung DE 101 502 86 genannten Verbindungen in Betracht. In einer besonders bevorzugten Ausführungsform kommen die in der deutschen Patentanmeldung DE 102 071 65 genannten Verbindungen in Betracht. In einer weiteren besonders bevorzugten Ausführungsform der vorliegenden Erfindung kommen die in der US 2003/0100442 A1 genannten phosphorhaltigen Chelatliganden in Betracht.

In einer weiteren besonders bevorzugten Ausführungsform der vorliegenden Erfindung kommen die in der nicht vorveröffentlichten deutschen Patentanmeldung Aktenzeichen DE 103 50 999.2 vom 30.10.2003 genannten phosphorhaltigen Chelatliganden in Betracht.

Solche Verbindungen (I) und deren Herstellung sind an sich bekannt.

Als phosphorhaltiger Ligand können auch Mischungen, enthaltend die Verbindungen (I), eingesetzt werden.

Die Hydrocyanierung kann gegebenenfalls auch in Gegenwart von zusätzlichen monodentaten phosphorhaltigen Liganden durchgeführt werden. Diese monodentaten phosphorhaltigen Liganden werden dabei vorzugsweise ausgewählt aus der Gruppe bestehend aus Phosphinen, Phosphiten, Phosphiniten und Phosphoniten.

Diese monodentaten phosphorhaltigen Liganden weisen vorzugsweise die Formel (II)

$$P(X^1R^1)(X^2R^2)(X^3R^3) \qquad (II)$$

auf.

Unter Verbindung (II) wird im Sinne der vorliegenden Erfindung eine einzelne Verbindung oder ein Gemisch verschiedener Verbindungen der vorgenannten Formel verstanden.

Erfindungsgemäß sind $X^1$, $X^2$, $X^3$ unabhängig voneinander Sauerstoff oder Einzelbindung. Falls alle der Gruppen $X^1$, $X^2$ und $X^3$ für Einzelbindungen stehen, so stellt Verbindung II ein Phosphin der Formel $P(R^1R^2R^3)$ mit den für $R^1$, $R^2$ und $R^3$ in dieser Beschreibung genannten Bedeutungen dar.

Falls zwei der Gruppen $X^1$, $X^2$ und $X^3$ für Einzelbindungen stehen und eine für Sauerstoff, so stellt Verbindung II ein Phosphinit der Formel $P(OR^1)(R^2)(R^3)$ oder $P(R^1)(OR^2)(R^3)$ oder $P(R^1)(R^2)(OR^3)$ mit den für $R^1$, $R^2$ und $R^3$ weiter unten genannten Bedeutungen dar.

Falls eine der Gruppen $X^1$, $X^2$ und $X^3$ für eine Einzelbindung steht und zwei für Sauerstoff, so stellt Verbindung II ein Phosphonit der Formel $P(OR^1)(OR^2)(R^3)$ oder $P(R^1)(OR^2)(OR^3)$ oder $P(OR^1)(R^2)(OR^3)$ mit den für $R^1$, $R^2$ und $R^3$ in dieser Beschreibung genannten Bedeutungen dar.

In einer bevorzugten Ausführungsform sollten alle der Gruppen $X^1$, $X^2$ und $X^3$ für Sauerstoff stehen, so dass Verbindung II vorteilhaft ein Phosphit der Formel $P(OR^1)(OR^2)(OR^3)$ mit den für $R^1$, $R^2$ und $R^3$ weiter unten genannten Bedeutungen darstellt.

Erfindungsgemäß stehen $R^1$, $R^2$, $R^3$ unabhängig voneinander für gleiche oder unterschiedliche organische Reste. Als $R^1$, $R^2$ und $R^3$ kommen unabhängig voneinander Alkylreste, vorzugsweise mit 1 bis 10 Kohlenstoffatomen, wie Methyl, Ethyl, n-Propyl, i-Propyl, n-Butyl, i-Butyl, s-Butyl, t-Butyl, Aryl-Gruppen, wie Phenyl, o-Tolyl, m-Tolyl, p-Tolyl, 1-Naphthyl, 2-Naphthyl, oder Hydrocarbyl, vorzugsweise mit 1 bis 20 Kohlenstoffatomen, wie 1,1'-Biphenol, 1,1'-Binaphthol in Betracht. Die Gruppen $R^1$, $R^2$ und $R^3$ können miteinander direkt, also nicht allein über das zentrale Phosphor-Atom, verbunden sein. Vorzugsweise sind die Gruppen $R^1$, $R^2$ und $R^3$ nicht miteinander direkt verbunden.

In einer bevorzugten Ausführungsform kommen als Gruppen $R^1$, $R^2$ und $R^3$ Reste ausgewählt aus der Gruppe bestehend aus Phenyl, o-Tolyl, m-Tolyl und p-Tolyl in Betracht. In einer besonders bevorzugten Ausführungsform sollten dabei maximal zwei der Gruppen $R^1$, $R^2$ und $R^3$ Phenyl-Gruppen sein.

In einer anderen bevorzugten Ausführungsform sollten dabei maximal zwei der Gruppen $R^1$, $R^2$ und $R^3$ o-Tolyl-

Gruppen sein.

Als besonders bevorzugte Verbindungen II können solche der Formel II a

$$(o\text{-Tolyl-O-})_w\ (m\text{-Tolyl-O-})_x\ (p\text{-Tolyl-O-})_y\ (\text{Phenyl-O-})_z\ P \qquad (II\ a)$$

eingesetzt werden, wobei w, x, y und z eine natürliche Zahl bedeuten, und folgende Bedingungen gelten: w + x + y + z = 3 und w, z ≤ 2.

Solche Verbindungen II a sind z.B. (p-Tolyl-O-)(Phenyl-O-)$_2$P, (m-Tolyi-O-)(Phenyl-O-)$_2$P, (o-Tolyl-O-) (Phenyl-O-)$_2$P, (p-Tolyl-O-)$_2$(Phenyl-O-)P, (m-Tolyl-O-)$_2$(Phenyl-O-)P, (o-Tolyl-O-)$_2$(Phenyl-O-)P, (m-Tolyl-O-)(p-Tolyl-O)(Phenyl-O-)P, (o-Tolyl-O-)(p-Tolyl-O-)(Phenyl-O-)P, (o-Tolyl-O-)(m-Tolyl-O-)(Phenyl-O-)P, (p-Tolyl-O-)$_3$P, (m-Tolyl-O-)(p-Tolyl-O-)$_2$P, (o-Tolyl-O-)(p-Tolyl-O-)$_2$P, (m-Tolyl-O-)$_2$(p-Toluyl-O-)P, (o-Tolyl-O-)$_2$(p-Tolyl-O-)P, (o-Tolyl-O-)(m-Tolyl-O-)(p-Tolyl-O)P, (m-Tolyl-O-)$_3$P, (o-Tolyl-O-)(m-Tolyl-O-)$_2$P (o-Tolyl-O-)$_2$(m-Tolyl-O-)P, oder Gemische solcher Verbindungen.

Gemische enthaltend (m-Tolyl-O-)$_3$P, (m -Tolyl-O-)$_2$(p-Tolyl-O-)P, (m-Tolyl-O-)(p-Tolyl-O-)$_2$P und (p-Tolyl-O-)$_3$P kann man beispielsweise durch Umsetzung eines Gemisches enthaltend m-Kresol und p-Kresol, insbesondere im Molverhältnis 2 : 1, wie es bei der destillativen Aufarbeitung von Erdöl anfällt, mit einem Phosphortrihalogenid, wie Phosphortrichlorid, erhalten.

In einer anderen, ebenfalls bevorzugten Ausführungsform kommen als phosphorhaltige Liganden die in der DE-A 199 53 058 näher beschriebenen Phosphite der Formel II b in Betracht:

$$P\ (O\text{-}R^1)_x\ (O\text{-}R^2)_y\ (O\text{-}R^3)_z\ (O\text{-}R^4)_p \qquad (II\ b)$$

mit

$R^1$: aromatischer Rest mit einem $C_1$-$C_{18}$-Alkylsubstituenten in o-Stellung zu dem Sauerstoffatom, das das Phosphoratom mit dem aromatischen System verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromatischen System verbindet, anellierten aromatischen System,

$R^2$: aromatischer Rest mit einem $C_1$-$C_{18}$-Alkylsubstituenten in m-Stellung zu dem Sauerstoffatom, das das Phosphoratom mit dem aromatischen System verbindet, oder mit einem aromatischen Substituenten in m-Stellung zu dem Sauerstoffatom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der aromatische Rest in o-Stellung zu dem Sauerstoffatom, das das Phosphoratom mit dem aromatischen System verbindet, ein Wasserstoffatom trägt,

$R^3$: aromatischer Rest mit einem $C_1$-$C_{18}$-Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aromatische Rest in o-Stellung zu dem Sauerstoffatom, das das Phosphoratom mit dem aromatischen System verbindet, ein Wasserstoffatom trägt,

$R^4$: aromatischer Rest, der in o-, m- und p-Stellung zu dem Sauerstoffatom, das das Phosphoratom mit dem aromatischen System verbindet, andere als die für $R^1$, $R^2$ und $R^3$ definierten Substituenten trägt, wobei der aromatische Rest in o-Stellung zu dem Sauerstoffatom, das das Phosphoratom mit dem aromatischen System verbindet, ein Wasserstoffatom trägt,

x : 1 oder 2,

y, z, p: unabhängig voneinander 0, 1 oder 2 mit der Maßgabe, dass x+y+z+p = 3.

Bevorzugte Phosphite der Formel II b sind der DE-A 199 53 058 zu entnehmen. Als Rest $R^1$ kommen vorteilhaft o-Tolyl-, o-Ethyl-phenyl-, o-n-Propyl-phenyl-, o-Isopropyl-phenyl-, o-n-Butyl-phenyl-, o-sek-Butyl-phenyl-, o-tert-Butyl-phenyl-, (o-Phenyl)-Phenyl- oder 1-Naphthyl- Gruppen in Betracht.

Als Rest $R^2$ sind m-Tolyl-, m-Ethyl-phenyl-, m-n-Propyl-phenyl-, m-Isopropyl-phenyl-, m-n-Butyl-phenyl-, m-sek-Butyl-phenyl-, m-tert-Butyl-phenyl-, (m-Phenyl)-Phenyl- oder 2-Naphthyl- Gruppen bevorzugt.

Als Rest $R^3$ kommen vorteilhaft p-Tolyl-, p-Ethyl-phenyl-, p-n-Propyl-phenyl-, p-Isopropyl-phenyl-, p-n-Butyl-phenyl-, p-sek-Butyl-phenyl-, p-tert-Butyl-phenyl- oder (p-Phenyl)-Phenyl-Gruppen in Betracht.

Rest $R^4$ ist bevorzugt Phenyl. Vorzugsweise ist p gleich null. Für die Indizes x, y, z und p in Verbindung II b ergeben sich folgende Möglichkeiten:

| x | y | z | p |
|---|---|---|---|
| 1 | 0 | 0 | 2 |
| 1 | 0 | 1 | 1 |
| 1 | 1 | 0 | 1 |
| 2 | 0 | 0 | 1 |
| 1 | 0 | 2 | 0 |
| 1 | 1 | 1 | 0 |
| 1 | 2 | 0 | 0 |
| 2 | 0 | 1 | 0 |
| 2 | 1 | 0 | 0 |

Bevorzugte Phosphite der Formel II b sind solche, in denen p gleich null ist sowie $R^1$, $R^2$ und $R^3$ unabhängig voneinander ausgewählt sind aus o-Isopropyl-phenyl, m-Tolyl und p-Tolyl, und $R^4$ Phenyl ist.

Besonders bevorzugte Phosphite der Formel II b sind solche, in denen $R^1$ der o-Isopropyl-phenyl-Rest, $R^2$ der m-Tolylrest und $R^3$ der p-Tolylrest ist mit den in der vorstehenden Tabelle genannten Indizes; außerdem solche, in denen $R^1$ der o-Tolylrest, $R^2$ der m-Tolylrest und $R^3$ der p-Tolylrest ist mit den in der Tabelle genannten Indizes; weiterhin solche, in denen $R^1$ der 1-Naphthylrest, $R^2$ der m-Tolylrest und $R^3$ der p-Tolylrest ist mit den in der Tabelle genannten Indizes; außerdem solche, in denen $R^1$ der o-Tolylrest, $R^2$ der 2-Naphthylrest und $R^3$ der p-Tolylrest ist mit den in der Tabelle genannten Indizes; und schließlich solche, in denen $R^1$ der o-Isopropyl-phenyl-Rest, $R^2$ der 2-Naphthylrest und $R^3$ der p-Tolylrest ist mit den in der Tabelle genannten Indizes; sowie Gemische dieser Phosphite.

Phosphite der Formel II b können erhalten werden, indem man

a) ein Phosphortrihalogenid mit einem Alkohol ausgewählt aus der Gruppe bestehend aus $R^1OH$, $R^2OH$, $R^3OH$ und $R^4OH$ oder deren Gemische umsetzt unter Erhalt eines Dihalogenophosphorigsäuremonoesters,

b) den genannten Dihalogenophosphorigsäuremonoester mit einem Alkohol ausgewählt aus der Gruppe bestehend aus $R^1OH$, $R^2OH$, $R^3OH$ und $R^4OH$ oder deren Gemische umsetzt unter Erhalt eines Monohalogenophosphorigsäurediesters und

c) den genannten Monohalogenophosphorigsäurediester mit einem Alkohol ausgewählt aus der Gruppe bestehend aus $R^1OH$, $R^2OH$, $R^3OH$ und $R^4OH$ oder deren Gemische umsetzt unter Erhalt eines Phosphits der Formel II b.

Die Umsetzung kann in drei getrennten Schritten durchgeführt werden. Ebenso können zwei der drei Schritte kombiniert werden, also a) mit b) oder b) mit c). Alternativ können alle der Schritte a), b) und c) miteinander kombiniert werden.

Dabei kann man geeignete Parameter und Mengen der Alkohole ausgewählt aus der Gruppe bestehend aus $R^1OH$, $R^2OH$, $R^3OH$ und $R^4OH$ oder deren Gemische durch einige einfache Vorversuche leicht ermitteln.

Als Phosphortrihalogenid kommen grundsätzlich alle Phosphortrihalogenide, vorzugsweise solche, in denen als Halogenid Cl, Br, I, insbesondere Cl, eingesetzt wird, sowie deren Gemische in Betracht. Es können auch Gemische verschiedener gleich oder unterschiedlich halogensubstituierter Phosphine als Phosphortrihalogenid eingesetzt werden. Besonders bevorzugt ist $PCl_3$. Weitere Einzelheiten zu den Reaktionsbedingungen bei der Herstellung der Phosphite II b und zur Aufarbeitung sind der DE-A 199 53 058 zu entnehmen.

Die Phosphite II b können auch in Form eines Gemisches verschiedener Phosphite II b als Ligand verwendet werden. Ein solches Gemisch kann beispielsweise bei der Herstellung der Phosphite II b anfallen.

In einer besonders bevorzugten Ausführungsform des erfindungsgemäßen Verfahrens ist der zusätzliche monodentate phosphorhaltige Ligand des Nickel(0)-Komplexes und/oder der zusätzliche monodentate freie phosphorhaltige Ligand ausgewählt aus Tritolylphosphit sowie den Phosphiten der Formel II b

$$P\,(O\text{-}R^1)_x\,(O\text{-}R^2)_y\,(O\text{-}R^3)_z\,(O\text{-}R^4)_p \qquad \text{(II b)}$$

worin $R^1$, $R^2$ und $R^3$ unabhängig voneinander ausgewählt sind aus o-Isopropyl-phenyl, m-Tolyl und p-Tolyl, $R^4$ Phenyl ist; x gleich 1 oder 2 ist, und y, z, p unabhängig voneinander 0,1 oder 2 sind mit der Maßgabe, dass x+y+z+p = 3 ist; und deren Mischungen.

Die beschriebenen Verbindungen I, II II a und II b sowie deren Herstellung sind an sich bekannt. Als zusätzlicher monodentater phosphorhaltiger Ligand neben einem Chelatliganden der Formel I oder einem Gemisch aus mehreren Chelatliganden der Formel I können auch Mischungen, enthaltend mindestens zwei der Verbindungen II, II a und II b, eingesetzt werden.

Die Hydrocyanierung kann in Gegenwart oder in Abwesenheit von einem Lösemittel durchgeführt werden. Wenn ein Lösemittel verwendet wird, so sollte das Lösemittel bei der gegebenen Reaktionstemperatur und dem gegebenen Reaktionsdruck flüssig und inert gegenüber den ungesättigten Verbindungen und dem mindestens einen Katalysator sein. Im Allgemeinen werden als Lösemittel Kohlenwasserstoffe, beispielsweise Benzol oder Xylol, oder Nitrile, beispielsweise Acetonitrile oder Benzonitril, verwendet. Vorzugsweise wird allerdings ein Ligand als Lösemittel verwendet. Ferner ist es möglich, mehrere, wie zwei oder drei, Lösemittel zu verwenden.

Die in dem erfindungsgemäßen Verfahren verwendeten Katalysatoren können beispielsweise durch reduktive Katalysatorsynthese hergestellt werden. Hierzu wird eine Nickel(II)-Quelle mit dem Ligand nach allgemein bekannten Verfahren, beispielsweise in US 6,127,567 und den dort zitierten Referenzen sowie den deutschen Patentanmeldungen DE 103 51 000.1, DE 103 51 002.8 und DE 103 51 003.6 der BASF AG beschrieben, zum Nickel(0)-Komplex umgesetzt.

Eine bevorzugte Ausführungsform der reduktiven Nickel-Katalysatorsynthese ist in der prioritätsälteren, nicht vorveröffentlichten deutschen Patentanmeldung DE 103 51 000.1 mit dem Titel "Verfahren zur Herstellung von Nickel(0)-Phosphorligand-Komplexen" der BASF AG beschrieben. Demgemäß erfolgt die Herstellung des Nickel(0)-Katalysators dadurch, dass man ein Nickel(II)-Ether-Addukt in Gegenwart mindestens eines Phosphor-haltigen Liganden reduziert. Das hierfür zu verwendende Nickel(II)-Ether-Addukt wird vorzugsweise dadurch hergestellt, dass man ein Nickelhalogenid in Wasser löst, mit einem Ether und einem organischen Nitril, gegebenenfalls unter Rühren, versetzt und anschließend Wasser und gegebenenfalls Ether entfernt. Das Nickel(II)-Ether-Addukt ist vorzugsweise wasserfrei und enthält in einer bevorzugten Ausführungsform ein Nickelhalogenid. Als Nickelhalogenid kommen Nickelchlorid, Nickelbromid und Nickeliodid in Frage. Bevorzugt ist Nickelchlorid. Das verwendete Nickel(II)-Ether-Addukt umfasst vorzugsweise einen Sauerstoff-haltigen, Schwefel-haltigen oder gemischt Sauerstoff-Schwefel-haltigen Ether. Dieser ist vorzugsweise ausgewählt aus der Gruppe, bestehend aus Tetrahydrofuran, Dioxan, Diethylether, Din-propylether, Diisopropylether, Di-n-butylether, Di-sec-butylether, Ethylenglykoldialkylether, Diethylenglykoldialkylether und Triethylenglykoldialkylether. Als Ethylenglykoldialkylether wird bevorzugt Ethylenglykoldimethylether (1,2-Dimethoxyethan, Glyme) und Ethylenglykoldiethylether verwendet. Als Diethylenglykoldialkylether wird bevorzugt Diethylenglykoldimethylether (Diglyme) verwendet. Als Triethylenglykoldialkylether wird bevorzugt Triethylenglykoldimethylether (Triglyme) verwendet.

Das zur Herstellung des Nickel(0)-Komplexes verwendete Reduktionsmittel ist vorzugsweise ausgewählt aus der Gruppe, bestehend aus Metallen, die elektropositiver als Nickel sind, Metallalkylen, elektrischem Strom, komplexen Hydriden und Wasserstoff.

In einer weiteren Ausführungsform kann der Nickel(0)-Katalysator durch ein Verfahren hergestellt werden, das in der prioritätsälteren, nicht vorveröffentlichten deutschen Patentanmeldung DE 103 51 002.8 mit dem Titel "Verfahren zur Herstellung von Nickel(0)-Phosphorligand-Komplexen" der BASF AG beschrieben ist. Demnach erfolgt die Herstellung des Nickel(0)-Komplexes dadurch, dass eine Nickel(II)-Quelle, die Nickelbromid, Nickeliodid oder Mischungen davon enthält, in Gegenwart mindestens eines Phosphor-haltigen Liganden reduziert wird. Dabei wird die Nickel(II)-Quelle vorzugsweise ohne vorherige besondere Trocknung verwendet. Es ist bevorzug, dass Die Herstellung erfolgt dabei vorzugsweise in einem Lösemittel, das ausgewählt ist aus der Gruppe, bestehend aus organischen Nitrilen, aromatischen oder aliphatischen Kohlenwasserstoffen oder Mischungen davon. Als Reduktionsmittel werden vorzugsweise Metalle, die elektropositiver als Nickel sind, verwendet. Gleichfalls ist auch die Vewendung von Metallalkylen, elektrischem Strom, komplexen Hydriden oder Wasserstoff möglich.

Darüber hinaus kann der in dem erfindungsgemäßen Verfahren verwendete Nickel(0)-Katalysator auch nach einem Verfahren hergestellt werden, das in der prioritätsälteren, nicht vorveröffentlichten deutschen Patentanmeldung DE 103 51 003.6 mit dem Titel "Einsatz von azeotrop-getrocknetem Nickel(II)-Halogeniden" der BASF AG beschrieben ist, hergestellt werden. Demnach erfolgt die Herstellung des Nickel(0)-Komplexes dadurch, dass ein durch Azeotropdestillation getrocknetes wasserhaltiges Nickel(II)-halogenid in Gegenwart mindestens eines Phosphor-haltigen Liganden reduziert wird. Das Nickel(II)-halogenid ist vorzugsweise ausgewählt aus der Gruppe, bestehend aus Nickel(II)-chlorid, Nickel(II)-bromid und Nickel(II)-iodid. Das durch Azeotropdestillation getrocknete Nickel(II)-halogenid wird vorzugsweise durch ein Verfahren zur Entfernung von Wasser aus den entsprechenden wasserhaltigen Nickel(II)-halogeniden hergestellt, wobei die Mischung mit einem Verdünnungsmittel versetzt wird, dessen Siedepunkt im Falle der Nichtazeotropbildung des genannten Verdünnungsmittels mit Wasser unter den Druckbedingungen der nachfolgend genannten Destillation höher ist als der Siedepunkt von Wasser und das an diesem Siedepunkt des Wassers flüssig vorliegt oder

das ein Azeotrop oder Heteroazeotrop mit Wasser unter den Druck- und Temperaturbedingungen der nachfolgend genannten Destillation bildet, und die Mischung, enthaltend das wasserhaltige Nickel(II)-halogenid und das Verdünnungsmittel, unter Abtrennung von Wasser oder des genannten Azeotrops oder des genannten Heteroazeotrops von dieser Mischung und unter Erhalt einer wasserfreien Mischung, enthaltend Nickel(II)-halogenid und das besagte Verdünnungsmittel destilliert wird. Das verwendete Verdünnungsmittel ist vorzugsweise ein organisches Verdünnungsmittel mit mindestens einer Nitrilgruppe. Die Reduktion zur Herstellung des entsprechenden Nickel(0)-Komplexes erfolgt vorzugsweise durch Metalle, die elektropositiver als Nickel sind. Alternativ ist auch die Verwendung von Metallalkylen, elektrischem Strom, Metallhydriden und Wasserstoff möglich.

Der in den Verfahren gemäß den zuvor beschriebenen Patentanmeldungen DE 103 51 000.1, DE 103 51 002.8 und DE 103 51 003.6 verwendete Ligand kann auch in einer Ligandlösung vorliegen, die bereits als Katalysatorlösung in Hydrocyanierungsreaktionen eingesetzt wurde und somit an Nickel(0) abgereichert ist.

Die Hydrocyanierung kann in jeder geeigneten, dem Fachmann bekannten Vorrichtung durchgeführt werden. Für die Reaktion kommen hierfür übliche Vorrichtungen in Betracht, wie sie beispielsweise in: Kirk-Othmer, Encyclopedia of Chemical Technology, 4. Ed., Vol. 20, John Wiley & Sons, New York, 1996, Seiten 1040 bis 1055 beschrieben sind, siehe Rührkesselreaktoren, Schlaufenreaktoren, Gasumlaufreaktoren, Blasensäulenreaktoren oder Rohrreaktoren, jeweils gegebenenfalls mit Vorrichtungen zur Abfuhr von Reaktionswärme. Die Reaktion kann in mehreren, wie zwei oder drei, Vorrichtungen durchgeführt werden.

In einer bevorzugten Ausführungsform des erfindungsgemäßen Verfahrens haben sich Reaktoren mit Rückvermischungscharakteristik oder Kaskaden von Reaktoren mit Rückvermischungscharakteristik als vorteilhaft erwiesen. Als besonders vorteilhaft haben sich Kaskaden aus Reaktoren mit Rückvermischungscharakteristik erwiesen, die in Bezug auf die Dosierung von Cyanwasserstoff in Querstromfahrweise betrieben werden.

Die Hydrocyanierung wird kontinuierlich vorzugsweise in einem oder mehreren gerührten Verfahrensschritten durchgeführt. Wenn eine Mehrzahl von Verfahrensschritten verwendet wird, so ist es bevorzugt, dass die Verfahrensschritte in Serie geschaltet sind. Dabei wird das Produkt von einem Verfahrensschritt direkt in den nächsten Verfahrensschritt überführt. Der Cyanwasserstoff kann direkt in den ersten Verfahrensschritt oder zwischen den einzelnen Verfahrensschritten zugeführt werden.

Die Reaktion wird vorzugsweise bei Drücken von 0,1 bis 500 MPa, besonders bevorzugt 0,5 bis 50 MPa, insbesondere 1 bis 5 MPa durchgeführt. Die Reaktion wird vorzugsweise bei Temperaturen von 273 bis 473 K, besonders bevorzugt 313 bis 423 K, insbesondere bei 333 bis 393 K durchgeführt. Dabei haben sich durchschnittliche mittlere Verweilzeiten der flüssigen Reaktorphase im Bereich von 0,001 bis 100 Stunden, vorzugsweise 0,05 bis 20 Stunden, besonders bevorzugt 0,1 bis 5 Stunden, pro Reaktor als vorteilhaft erwiesen.

Die Reaktion kann in einer Ausführungsform in flüssiger Phase in Gegenwart einer Gasphase und gegebenenfalls einer festen suspendierten Phase ausgeführt werden. Dabei können die Ausgangsstoffe Cyanwasserstoff und 1,3-Butadien jeweils flüssig oder gasförmig zudosiert werden.

Die Reaktion kann in einer weiteren Ausführungsform in flüssiger Phase durchgeführt werden, wobei der Druck im Reaktor so bemessen ist, dass alle Edukte wie 1,3-Butadien, Cyanwasserstoff und der mindestens eine Katalysator flüssig zudosiert werden und in der Reaktionsmischung in flüssiger Phase vorliegen. Dabei kann eine feste suspendierte Phase im Reaktionsgemisch vorliegen, die auch zusammen mit dem mindestens einen Katalysator zudosiert werden kann, beispielsweise bestehend aus Abbauprodukten des Katalysatorsystems, enthaltend unter anderem Nickel(II)-Verbindungen.

In einer bevorzugten Ausführungsform ist das erfindungsgemäße Verfahren dadurch gekennzeichnet, dass die kontinuierliche Hydrocyanierung in Gegenwart mindestens einer Lewis-Säure durchgeführt wird.

Erfindungsgemäß wurde gefunden, dass bei Einsatz eines Überschusses an 1,3-Butadien die Gegenwart einer Lewis-Säure nicht zur, für Monophosphitkomplexe literaturbekannten Bildung von Dintirilen in Form von Methylglutardinitril führt. Die Ergebnisse, die mit dem erfindungsgemäßen Verfahren erhalten werden, sind denen ohne Zusatz an Lewis-Säure vergleichbar.

Das erfindungsgemäße Verfahren ist in einer bevorzugten Ausführungsform durch die folgenden Verfahrensschritte gekennzeichnet:

(a) kontinuierliche Hydrocyanierung von 1,3-Butadien in Gegenwart mindestens eines Nickel(0)-Katalysators mit Chelatliganden und gegebenenfalls in Gegenwart mindestens einer Lewis-Säure, wobei 1,3-Butadien und Cyanwasserstoff in einem Verhältnis von 1,6 bis 1,1 zu 1, vorzugsweise 1,6 bis 1,3 zu 1, verwendet werden und eine Mischung 1 erhalten wird, die 3-Pentennitril und 2-Methyl-3-butennitril enthält;

(c) kontinuierliche Isomerisierung von 2-Methyl-3-butennitril, das in der Mischung 1 enthalten ist, an mindestens einem gelösten oder dispergierten Isomerisierungskatalysator zu 3-Pentennitril, wobei eine Mischung 2 resultiert.

Erfindungsgemäß führt man die Isomerisierung in Gegenwart eines Systems, enthaltend

a) Nickel(0),

b) eine Nickel(0) als Ligand komplexierende, dreibindigen Phosphor enthaltende Verbindung und gegebenenfalls

c) eine Lewis-Säure

durch.

Die Herstellung von Nickel(0) enthaltenden Katalysatorsystemen kann nach an sich bekannten Verfahren erfolgen.

Als Liganden für den Isomerisierungskatalysator können die gleichen Phosphorhaltigen Liganden wie für den Hydrocyanierungskatalysator verwendet werden. Somit ist in einer bevorzugten Ausführungsform des erfindungsgemäßen Verfahrens der in Verfahrensschritt (c) verwendete Isomerisierungskatalysator der in Verfahrensschritt (a) verwendete Nickel(0)-Katalysators mit Chelatliganden.

Weiterhin enthält das System gegebenenfalls eine Lewis-Säure. Die Verwendung einer Lewis-Säure in der Isomerisierung von 2-Methyl-3-butennitril führt zu einer Erhöhung der Reaktionsgeschwindigkeit. Hierdurch wird die Absenkung der Reaktionstemperatur ermöglicht und somit die thermische Belastung des Katalysators verringert.

Im Sinne der vorliegenden Erfindung wird unter einer Lewis-Säure eine einzelne Lewis-Säure oder ein Gemisch aus mehreren, wie zwei, drei oder vier Lewis-Säuren, verstanden.

Als Lewis-Säure kommen dabei anorganische oder organische Metall-Verbindungen in Betracht, in denen das Kation ausgewählt ist aus der Gruppe, bestehend aus Scandium, Titan, Vanadium, Chrom, Mangan, Eisen, Kobalt, Kupfer, Zink, Bor, Aluminium, Yttrium, Zirkonium, Niob, Molybdän, Cadmium, Rhenium und Zinn. Beispiele sind $ZnBr_2$, $ZnI_2$, $ZnCl_2$, $ZnSO_4$, $CuCl_2$, CuCl, $Cu(O_3SCF_3)_2$, $CoCl_2$, $CoI_2$, $FeI_2$, $FeCl_3$, $FeCl_2$, $FeCl_2(THF)_2$, $TiCl_4(THF)_2$, $TiCl_4$, $TiCl_3$, $ClTi(O\text{-}i\text{-}Propyl)_3$, $MnCl_2$, $ScCl_3$, $AlCl_3$, $(C_8H_{17})AlCl_2$, $(C_8H_{17})_2AlCl$, $(i\text{-}C_4H_9)_2AlCl$, $(C_6H_5)_2AlCl$, $(C_6H_5)AlCl_2$, $ReCl_5$, $ZrCl_4$, $NbCl_5$, $VCl_3$, $CrCl_2$, $MoCl_5$, $YCl_3$, $CdCl_2$, $LaCl_3$, $Er(O_3SCF_3)_3$, $Yb(O_2CCF_3)_3$, $SmCl_3$, $B(C_6H_5)_3$, $TaCl_5$, wie beispielsweise in US 6,127,567, US 6,171,996 und US 6,380,421 beschrieben. Weiterhin kommen in Betracht Metallsalze, wie $ZnCl_2$, $CoI_2$ und $SnCl_2$ sowie organometallische Verbindungen, wie $RAlCl_2$, $R_2AlCl$, $RSnO_3SCF_3$ und $R_3B$, wobei R eine Alkyl- oder Aryl-Gruppe ist, wie beispielsweise in US 3,496,217, US 3,496,218 und US 4,774,353 beschrieben. Weiterhin können gemäß US 3,773,809 als Promotor ein Metall in kationischer Form, ausgewählt aus der Gruppe, bestehend aus Zink, Cadmium, Beryllium, Aluminium, Gallium, Indium, Thallium, Titan, Zirkonium, Hafnium, Erbium, Germanium, Zinn, Vanadium, Niob, Scandium, Chrom, Molybdän, Wolfram, Mangan, Rhenium, Palladium, Thorium, Eisen und Kobalt, vorzugsweise Zink, Cadmium, Titan, Zinn, Chrom, Eisen und Kobalt, eingesetzt werden, wobei der anionische Teil der Verbindung ausgewählt sein kann aus der Gruppe, bestehend aus Halogeniden, wie Fluorid, Chlorid, Bromid und Jodid, Anionen niedriger Fettsäuren mit von 2 bis 7 Kohlenstoffatomen, $HPO_3^{2-}$, $H_3PO^{2-}$, $CF_3COO^-$, $C_7H_{15}OSO_2^-$ oder $SO_4^{2-}$. Weiterhin sind aus US 3,773,809 als geeignete Promotoren Borhydride, Organoborhydride und Borsäureester der Formel $R_3B$ und $B(OR)_3$, wobei R ausgewählt ist aus der Gruppe, bestehend aus Wasserstoff, Aryl-Radikalen mit zwischen 6 und 18 Kohlenstoff-Atomen, mit Alkyl-Gruppen mit 1 bis 7 Kohlenstoff-Atomen substituierten Aryl-Radikalen und mit Cyanosubstituierten Alkyl-Gruppen mit 1 bis 7 Kohlenstoff-Atomen substituierten Aryl-Radikalen, vorteilhaft Triphenylbor, genannt. Weiterhin können, wie in US 4,874,884 beschrieben, synergistisch wirksame Kombinationen von Lewis-Säuren eingesetzt werden, um die Aktivität des Katalysatorsystems zu erhöhen. Geeignete Promotoren können beispielsweise aus der Gruppe bestehend aus $CdCl_2$, $FeCl_2$, $ZnCl_2$, $B(C_6H_5)_3$ und $(C_6H_5)_3SnX$, mit $X=CF_3SO_3$, $CH_3C_6H_4SO_3$ oder $(C_6H_5)_3BCN$ ausgewählt werden, wobei für das Verhältnis von Promotor zu Nickel ein Bereich von vorzugsweise etwa 1:16 bis etwa 50:1 genannt ist.

Im Sinne der vorliegenden Erfindung umfasst der Begriff Lewis-Säure auch die in US 3,496,217, US 3,496,218, US 4,774,353, US 4,874,884, US 6,127,567, US 6,171,996 und US 6,380,421 genannten Promotoren.

Als besonders bevorzugte Lewis-Säuren kommen unter den genannten insbesondere Metallsalze, besonders bevorzugt Metallhalogenide, wie Fluoride, Chloride, Bromide, Jodide, insbesondere Chloride, in Betracht, von denen wiederum Zinkchlorid, Eisen-(II)-Chlorid und Eisen-(III)-chlorid besonders bevorzugt sind.

Die Isomerisierung kann in Gegenwart eines flüssigen Verdünnungsmittels,

- beispielsweise eines Kohlenwasserstoffs, wie Hexan, Heptan, Oktan, Cyclohexan, Methylcyclohexan, Benzol, Decahydronaphthalin
- beispielsweise eines Ethers, wie Diethylether, Tetrahydrofuran, Dioxan, Glykoldimethylether, Anisol,
- beispielsweise eines Esters, wie Ethylacetat, Methylbenzoat, oder
- beispielsweise eines Nitrils, wie Acetonitril, Benzonitril, oder
- Gemischen solcher Verdünnungsmittel durchgeführt werden.

In einer besonders bevorzugten Ausführungsform kommt eine Isomerisierung in Abwesenheit eines solchen flüssigen Verdünnungsmittels in Betracht.

Weiterhin hat es sich als vorteilhaft erwiesen, wenn die Isomerisierung und/oder die Hydrocyanierung in nichtoxidierend wirkender Atmosphäre, beispielsweise unter einer Schutzgasatmosphäre aus Stickstoff oder einem Edelgas, beispielsweise Argon, durchgeführt wird. Dabei werden die Isomerisierung und/oder die Hydrocyanierung vorzugsweise unter Feuchtigkeitsausschluss durchgeführt.

Die Isomerisierung kann in jeder geeigneten, dem Fachmann bekannten Vorrichtung durchgeführt werden. Für die Isomerisierung kommen somit übliche Vorrichtungen in Betracht, wie sie beispielsweise in: Kirk-Othmer, Encyclopedia of Chemical Technology, 4. Ed., Vol. 20, John-Wiley & Sons, New York, 1996, Seiten 1040 bis 1055 beschrieben sind, wie Rührkesselreaktoren, Schlaufenreaktoren, Gasumlaufreaktoren, Blasensäulenreaktoren oder Rohrreaktoren. Die Reaktion kann in mehreren, wie zwei oder drei Vorrichtungen durchgeführt werden.

In einer bevorzugten Ausführungsform des erfindungsgemäßen Verfahrens wird die Isomerisierung in einem kompartimentiertem Rohrreaktor durchgeführt.

In einer weiteren bevorzugten Ausführungsform des erfindungsgemäßen Verfahrens wird die Isomerisierung in mindestens zwei in Serie geschalteten Reaktoren durchgeführt, wobei der erste Reaktor im Wesentlichen Rührkesselcharakteristik aufweist und der zweite Reaktor so ausgeführt ist, dass er im Wesentlichen Rohrcharakteristik aufweist.

In einer besonders bevorzugten Ausführungsform des erfindungsgemäßen Verfahrens wird die Isomerisierung in einem Reaktor durchgeführt, wobei der Reaktor die Charakteristik einer Rührkesselkaskade aufweist, die 2 bis 20 Rührkesseln, insbesondere 3 bis 10 Rührkesseln, entspricht.

Die Isomerisierung wird vorzugsweise bei einem absoluten Druck von 0,1 mbar bis 100 bar, besonders bevorzugt 1 mbar bis 16 bar, insbesondere 10 mbar bis 6 bar, durchgeführt. Die Temperatur beträgt dabei vorzugsweise 80 bis 125 °C, besonders bevorzugt 85 bis 120 °C, insbesondere 90 bis 115 °C.

In einer besonders bevorzugten Ausführungsform des erfindungsgemäßen Verfahrens wird zwischen dem Verfahrensschritt (a) und (c) folgender Verfahrensschritt (b) durchlaufen:

(b) destillative Entfernung von 1,3-Butadien aus der Mischung 1.

Erfindungsgemäß wurde festgestellt, dass 1,3-Butadien als Inhibitor in der Isomerisierung von 2-Methyl-3-butennitril wirkt. Daher ist es von der Verfahrensführung besonders bevorzugt, dass das 1,3-Butadien vor der Isomerisierung entfernt wird.

In einer besonders bevorzugten Ausführungsform des erfindungsgemäßen Verfahrens wird das durch das erfindungsgemäße Verfahren in Verfahrensschritt (c) erhaltene 3-Pentennitril, gegebenenfalls nach einer Isomersierung des parallel erhaltenen 2-Methyl-3-butennitrils gemäß oben beschriebener Ausführungsform, einer weiteren Hydrocyanierung in Gegenwart mindestens einer Lewis-Säure zu Adipodinitril unterzogen. Die hierbei anzuwendenden Verfahrensbedingungen sind an sich bekannt. Auch in dieser Hydrocyanierung wird ein Nickel(0)-Katalysator mit phosphorhaltigen Liganden verwendet. Daher bietet sich für ein ökonomisches Verfahren an, die in den jeweiligen Hydrocyanierungen verwendeten Katalysatorkreisläufe miteinander zu verschalten, so dass dass auch in der zweiten Hydrocyanierung dasselbe Katalysatorsystem wie in der ersten Hydrocyanierung verwendet wird. Eine entsprechende Verfahrensweise ist in der DE-A-102 004 004 682 beschrieben. Da das erfindungsgemäße Verfahren es nun ermöglicht, eine Hydrocyanierung von 1,3-Butadien mit Lewis-Säure durchzuführen, ist eine wie bisher vorgesehene aufwendige quantitative Abtrennung der Lewis-Säure aus der zweiten Hydrocyanierung von 3-Pentennitril vor dem Einsatz in der ersten Hydrocyanierung von 1,3-Butadien bei Einsatz eines Überschusses an 1,3-Butadien nicht notwendig. Somit genügt in der vorliegenden Erfindung vorzugsweise eine einfache Extraktion zur Dinitrilabtrennung nach der zweiten Hydrocyanierung.

Falls eine Isomerisierung durchgeführt wird, so ist besonders bevorzugt, dass in der ersten und der zweiten Hydrocyanierung wie in der Isomerisierung das gleiche Katalysatorsystem verwendet wird. Durch die Möglichkeit, eine Lewis-Säure in der ersten Hydrocyanierung ohne Selektivitätsverlust zu zulassen, kann die Katalysatorextraktion verkleinert werden.

Die vorliegende Erfindung wird anhand der nachfolgenden Ausführungsbeispiele näher erläutert.

**Beispiel: Kontinuierliche Hydrocyanierung von BD zu 2M3BN/3PN**

In den Beispielen werden folgende Abkürzungen verwendet:

| | |
|---|---|
| HCN: | Cyanwasserstoff |
| 3PN: | 3-Pentennitril |
| MGN: | Methyglutardinitril |
| 2M3BN: | 2-Methyl-3-butennitril |
| BD: | 1,3-Butadien |
| THF: | Tetrahydrofuran |

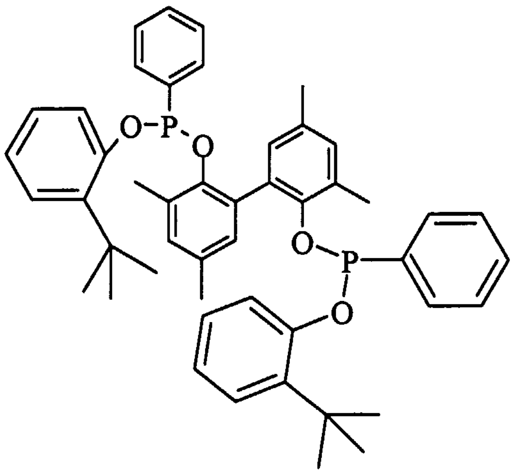

Ligand 1

Beispiel 1: (Verhältnis BD/HCN = 1/1)

1,65 mol 1,3-Butadien, 1,65 mol HCN und 4 mmol Ni in Form einer Katalysatorlösung, bestehend aus 1 mmol Ni(0), 2 mmol Ligand 1 und 4 mmol m-/p-Tolylphosphit, gelöst in 3-Pentennitril, werden pro Stunde in einen Druckreaktor eingespeist (Druck: 15 bar, Temperatur innen 90 °C, Verweilzeit: 40 Min/Reaktor). Der HCN-Umsatz ist nach Maßanalyse quantitativ (Titration nach Vollhardt). Man bestimmt das Verhältnis 2M3BN/3PN des Reaktionsaustrages GC-Chromatographisch (GC-Flächenprozent). Das Verhältnis 2M3BN/3PN beträgt 1/0.76. Die Ausbeute bezogen auf HCN beträgt: 87.7 % Pentennitril, 10.4 % MGN, 1.7 % 2M2BN.

Beispiel 2: (Verhältnis BD/HCN = 1.25/1)

1,9 mol 1,3-Butadien, 1,5 mol HCN und 5.4 mmol Ni in Form einer Katalysatorlösung, bestehend aus 1 mmol Ni(0), 2 mmol Ligand 1 und 4 mmol m-/p-Tolylphosphit, gelöst in 3-Pentennitril, werden pro Stunde in einen Druckreaktor eingespeist (Druck: 15 bar, Temperatur innen 90 °C, Verweilzeit: 40 Min/Reaktor). Der HCN-Umsatz ist nach Maßanalyse quantitativ (Titration nach Vollhard). Man bestimmt das Verhältnis 2M3BN/3PN des Reaktionsaustrages GC-Chromatographisch (GC-Flächenprozent). Das Verhältnis 2M3BN/3PN beträgt1/0.73. Die Ausbeute bezogen auf HCN beträgt 94.6 % Pentennitril, 4.0 MGN, 1.3 % 2M2BN.

Beispiel 3: (Verhältnis BD/HCN = 1.5/1)

2,45 mol 1,3-Butadien, 1,65 mol HCN und 4 mmol Ni in Form einer Katalysatorlösung, bestehend aus 1 mmol Ni(0), 2 mmol Ligand 1 und 4 mmol m-/p-Tolylphosphit, gelöst in 3-Pentennitril, werden pro Stunde in einen Druckreaktor eingespeist (Druck: 15 bar, Temperatur innen 90 °C, Verweilzeit: 33 Min/Reaktor). Der HCN-Umsatz ist nach Maßanalyse quantitativ (Titration nach Vollhard). Man bestimmt das Verhältnis 2M3BN/3PN des Reaktionsaustrages GC-Chromatographisch (GC-Flächenprozent). Das Verhältnis 2M3BN/3PN beträgt 1/0.73. Die Ausbeute bezogen auf HCN beträgt 95.9 % Pentennitril, 1.3 MGN, 2.1 % 2M2BN.

**Beispiel: Synthese und Isomerisierung eines kontinuierlichen Reaktoraustrages**

Beispiel 4: (ohne Butadienabtrennung)

Aus einer Katalysatorlösung, bestehend aus 0.56 % Ni(0), 62.2 % 3PN und 37,24 % Ligand 1, werden 2 mmol Ni(0) entnommen, mit 611 mmol BD versetzt, bei 25 °C in einen Glasautoklaven gefüllt und auf 90 °C erwärmt. Über 81 Min. werden nun 400 mmol HCN in THF zudosiert, weitere 60 Min. bei 90 °C nachgerührt und eine Probe genommen. Der HCN-Umsatz ist quantitativ (Maßanalyse nach Vollhard). Man bestimmt zudem das Verhältnis 2M3BN/3PN GC-Chromatographisch (GC-Flächenprozent). Das Verhältnis 2M3BN/3PN beträgt 1/1.36.

Anschließend wird der gesamte Ansatz auf 115 °C für 120 Min. erwärmt, um 2M3BN direkt zu 3PN zu isomerisieren. Man nimmt eine Probe und bestimmt das Verhältnis 2M3BN/3PN GC-Chromatographisch (GC-Flächenprozent). Das Verhältnis 2M3BN/ 3PN beträgt 1/3.3.

Beispiel 5: (mit Butadienabtrennung)

Aus einer Katalysatorlösung, bestehend aus 0.56 % Ni(0), 62.2 % 3PN und 37,24 % Ligand 1, werden 2 mmol Ni(0) entnommen, mit 705 mmol BD versetzt, bei 25 °C in einen Glasautoklaven gefüllt und auf 90 °C erwärmt. Über 72 Min. werden nun 411 mmol HCN in THF zudosiert, weitere 60 Min. bei 90 °C nachgerührt und eine Probe genommen. Der HCN-Umsatz ist quantitativ (Maßanalyse nach Vollhard). Man destilliert nun bei 100 mbar und 50 °C das überschüssige BD ab und bestimmt das Verhältnis 2M3BN/3PN GC-Chromatographisch (GC-Flächenprozent). Das Verhältnis 2M3BN/3PN beträgt 1/1.4.

Anschließend wird der Gesamte Ansatz auf 115 °C für 120 Min. erwärmt, um 2M3BN direkt zu 3PN zu isomerisieren. Man nimmt eine Probe und bestimmt das Verhältnis 2M3BN/3PN GC-Chromatographisch (GC-Flächenprozent). Das Verhältnis 2M3BN/3PN beträgt 1/13.2.

Beispiel 6: (ohne Butadienabtrennung)

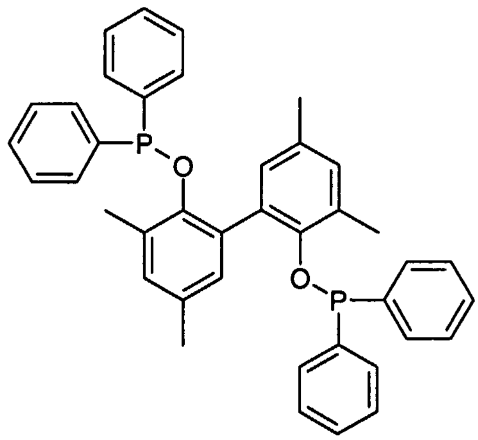

Ligand 2

6 mmol Ligand 2, werden mit 2 mmol $Ni(COD)_2$ und 581 mmol BD in THF versetzt, bei 25 °C in einen Glasautoklaven gefüllt und auf 90 °C erwärmt. Über 85 Min. werden nun 407 mmol HCN in THF zudosiert, weitere 60 Min. bei 90 °C nachgerührt und eine Probe genommen. Der HCN-Umsatz ist quantitativ (Maßanalyse nach Vollhard). Man bestimmt zudem das Verhältnis 2M3BN/3PN GC-Chromatographisch (GC-Flächenprozent). Das Verhältnis 2M3BN/3PN beträgt 1/0.55.

Anschließend wird der Gesamte Ansatz auf 115 °C für 120 Min. erwärmt, um 2M3BN direkt zu 3PN zu isomerisieren. Man nimmt eine Probe und bestimmt das Verhältnis 2M3BN/3PN GC-Chromatographisch (GC-Flächenprozent). Das Verhältnis 2M3BN/ 3PN beträgt 1/1.2.

Beispiel 7: (mit Butadienabtrennung)

6 mmol Ligand 2, werden mit 2 mmol $Ni(COD)_2$ und 583 mmol BD in THF versetzt, bei 25 °C in einen Glasautoklaven gefüllt und auf 90 °C erwärmt. Über 105 Min. werden nun 411 mmol HCN in THF zudosiert, weitere 60 Min. bei 90 °C nachgerührt und eine Probe genommen. Der HCN-Umsatz ist quantitativ (Maßanalyse nach Vollhard). Man destilliert nun bei 100 mbar und 50 °C das überschüssige BD ab und bestimmt das Verhältnis 2M3BN/3PN GC-Chromatographisch (GC-Flächenprozent). Das Verhältnis 2M3BN3PN beträgt 1/0.6.

Anschließend wird der gesamte Ansatz auf 115 °C für 120 Min. erwärmt, um 2M3BN, direkt zu 3PN zu isomerisieren. Man nimmt eine Probe und bestimmt das Verhältnis 2M3BN/3PN GC-Chromatographisch (GC-Flächenprozent). Das Verhältnis 2M3BN/3PN beträgt 1/8.9.

Beispiel 8: (ohne Butadienabtrennung)

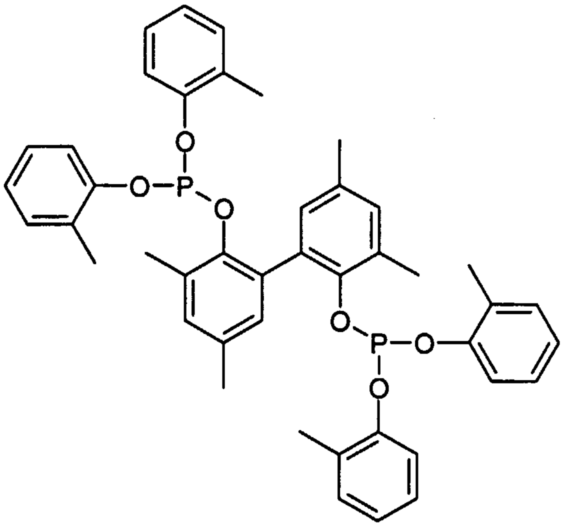

Ligand 3

6.3 mmol Ligand 3, werden mit 2 mmol $Ni(COD)_2$ und 581 mmol BD in THF versetzt, bei 25 °C in einen Glasautoklaven gefüllt und auf 90 °C erwärmt. Über 85 Min. werden nun 407 mmol HCN in THF zudosiert, weitere 60 Min. bei 90 °C nachgerührt und eine Probe genommen. Der HCN-Umsatz ist quantitativ (Maßanalyse nach Vollhard). Man destilliert nun bei 100 mbar und 50 °C das überschüssige BD ab und bestimmt das Verhältnis 2M3BN/3PN GC-Chromatographisch (GC-Flächenprozent). Das Verhältnis 2M3BN3PN beträgt 1/2.4.

Anschließend wird der gesamte Ansatz auf 115 °C für 120 Min. erwärmt, um 2M3BN direkt zu 3PN zu isomerisieren. Man nimmt eine Probe und bestimmt das Verhältnis 2M3BN/3PN GC-Chromatographisch (GC-Flächenprozent). Das Verhältnis 2M3BN/ 3PN beträgt 1/4.3.

Beispiel 9: (mit Butadienabtrennung)

6 mmol Ligand 3, werden mit 2 mmol $Ni(COD)_2$ und 606 mmol BD in THF versetzt, bei 25 °C in einen Glasautoklaven gefüllt und auf 90 °C erwärmt. Über 76 Min. werden nun 400 mmol HCN in THF zudosiert, weitere 60 Min. bei 90 °C nachgerührt und eine Probe genommen. Der HCN-Umsatz ist quantitativ (Maßanalyse nach Vollhard). Man destilliert nun bei 100 mbar und 50 °C das überschüssige BD ab und bestimmt das Verhältnis 2M3BN/3PN GC-Chromatographisch (GC-Flächenprozent). Das Verhältnis 2M3BN3PN beträgt 1/2.8.

Anschließend wird der Gesamte Ansatz auf 115 °C für 120 Min. erwärmt, um 2M3BN direkt zu 3PN zu isomerisieren. Man nimmt eine Probe und bestimmt das Verhältnis 2M3BN/3PN GC-Chromatographisch (GC-Flächenprozent). Das Verhältnis 2M3BN/3PN beträgt 1/20.

Beispiel 10: (ohne Butadienabtrennung)

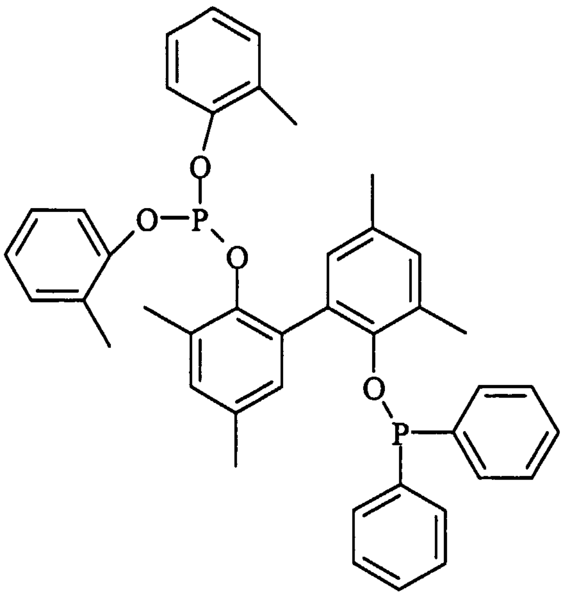

Ligand 4

2.6 mmol Ligand 4, werden mit 0.84mmol $Ni(COD)_2$ und 612 mmol BD in THF versetzt, bei 25 °C in einen Glasautoklaven gefüllt und auf 90 °C erwärmt. Über 80 Min. werden nun 389 mmol HCN in THF zudosiert, weitere 60 Min. bei 90 °C nachgerührt und eine Probe genommen. Der HCN-Umsatz ist quantitativ (Maßanalyse nach Vollhard). Man destilliert nun bei 100 mbar und 50 °C das überschüssige BD ab und bestimmt das Verhältnis 2M3BN/3PN GC-Chromatographisch (GC-Flächenprozent). Das Verhältnis 2M3BN3PN beträgt 1/1.3.

Anschließend wird der Gesamte Ansatz auf 115 °C für 120 Min. erwärmt, um 2M3BN direkt zu 3PN zu isomerisieren. Man nimmt eine Probe und bestimmt das Verhältnis 2M3BN/3PN GC-Chromatographisch (GC-Flächenprozent). Das Verhältnis 2M3BN/3PN beträgt 1/5.2.

Beispiel 11: (mit Butadienabtrennung)

2.7 mmol Ligand 3, werden mit 0.89 mmol $Ni(COD)_2$ und 655 mmol BD in THF versetzt, bei 25 °C in einen Glasautoklaven gefüllt und auf 90 °C erwärmt. Über 70 Min. werden nun 414 mmol HCN in THF zudosiert, weitere 60 Min. bei 90 °C nachgerührt und eine Probe genommen. Der HCN-Umsatz ist quantitativ (Maßanalyse nach Vollhard). Man destilliert nun bei 100 mbar und 50 °C das überschüssige BD ab und bestimmt das Verhältnis 2M3BN/3PN GC-Chromatographisch (GC-Flächenprozent). Das Verhältnis 2M3BN3PN beträgt 1.3/1.

Anschließend wird der Gesamte Ansatz auf 115 °C für 65 Min. erwärmt, um 2M3BN direkt zu 3PN zu isomerisieren. Man nimmt eine Probe und bestimmt das Verhältnis 2M3BN/3PN GC-Chromatographisch (GC-Flächenprozent). Das Verhältnis 2M3BN/3PN beträgt 1/20.

**Beispiel: Hydrocyanierung von BD in Gegenwart einer LS**

Beispiel 12:

1.64 mmol Ligand 3, werden mit 0.55 mol $Ni(COD)_2$, 0.55 mmol $ZnCl_2$ und 380 mmol BD in THF versetzt, bei 25 °C in einen Glasautoklaven gefüllt und auf 90 °C erwärmt. Über 100 Min. werden nun 253 mmol HCN in THF zudosiert, weitere 60 Min. bei 90 °C nachgerührt und eine Probe genommen. Der HCN-Umsatz ist quantitativ (Maßanalyse nach Vollhard). Man bestimmt zudem das Verhältnis 2M3BN/3PN GC-Chromatographisch (GC-Gewichtsprozent, int. Standart: Ethylbenzol). Das Verhältnis 2M3BN/3PN beträgt 1/1.1. Es bildeten sich 0.2 % MGN.

Beispiel 13:

1.72 mmol Ligand 4, werden mit 0.56 mol $Ni(COD)_2$, 0.57 mmol $ZnCl_2$ und 402 mmol BD in THF versetzt, bei 25 °C in einen Glasautoklaven gefüllt und auf 90 °C erwärmt. Über 52 Min. werden nun 259 mmol HCN in THF zudosiert, weitere 60 Min. bei 90 °C nachgerührt und eine Probe genommen. Der HCN-Umsatz ist quantitativ (Maßanalyse nach Vollhard). Man bestimmt zudem das Verhältnis 2M3BN/3PN GC-Chromatographisch (GC-Gewichtsprozent, int. Standart: Ethylbenzol). Das Verhältnis 2M3BN/3PN beträgt 1.5/1. Es bildete sich kein MGN.

## Patentansprüche

1. Verfahren zur kontinuierlichen Hydrocyanierung von 1,3-Butadien in Gegenwart mindestens eines Katalysators, **dadurch gekennzeichnet, dass** man als Katalysatoren Nickel(0)-Katalysatoren, die mit phosphorhaltigen Chelatliganden stabilisiert sind, 1,3-Butadien und Cyanwasserstoff in einem molaren Verhältnis von 1,6 bis 1,1 zu 1 verwendet und dass die kontinuierliche Hydrocyanierung zusätzlich in Gegenwart mindestens einer Lewis-Säure durchgeführt wird.

2. Verfahren nach Anspruch 1, **dadurch gekennzeichnet, dass** der Nickel(0)-Katalysator mit phosphorhaltigen Chelatliganden abgesättigt ist, wobei die phosphorhaltigen Chelatliganden ausgewählt sind aus der Gruppe, bestehend aus bidentaten Phosphiten, Phosphinen, Phosphoniten, Phosphiniten und Phosphinitphosphiten.

3. Verfahren nach Anspruch 1 oder 2, **gekennzeichnet durch** die folgenden Verfahrensschritte:

   (a) kontinuierliche Hydrocyanierung von 1,3-Butadien in Gegenwart mindestens eines Nickel(0)-Katalysators mit Chelatliganden und gegebenenfalls in Gegenwart mindestens einer Lewis-Säure, wobei 1,3-Butadien und Cyanwasserstoff in einem molaren Verhältnis von 1,6 bis 1,1 zu 1 verwendet werden und eine Mischung 1 erhalten wird, die 3-Pentennitril und 2-Methyl-3-butennitril enthält;
   (c) kontinuierliche Isomerisierung von 2-Methyl-3-butennitril, das in der Mischung 1 enthalten ist, an mindestens einem gelösten oder dispergierten Isomerisierungskatalysator zu 3-Pentennitril, wobei eine Mischung 2 resultiert.

4. Verfahren nach Anspruch 3, **dadurch gekennzeichnet, dass** das in Verfahrensschritt (c) erhaltene 3-Pentennitril in Gegenwart mindestens eines Nickel(0)-Katalysators mit phosphorhaltigen Liganden hydrocyaniert wird.

5. Verfahren nach Anspruch 3 oder 4, **dadurch gekennzeichnet, dass** die Isomerisierung in Verfahrensschritt (c) durch Erhitzen der Mischung 1 auf 80 bis 125 °C erfolgt.

6. Verfahren nach einem der Ansprüche 3 bis 5, **dadurch gekennzeichnet, dass** die in Verfahrensschritt (c) durchgeführte kontinuierliche Isomerisierung in Gegenwart mindestens einer Lewis-Säure durchgeführt wird.

7. Verfahren nach einem der Ansprüche 3 bis 6, **dadurch gekennzeichnet, dass** zwischen Verfahrensschritt (a) und Verfahrensschritt (c) folgender Verfahrensschritt (b) durchlaufen wird:

   (b) destillative Entfernung von 1,3-Butadien aus der Mischung 1.

8. Verfahren nach einem der Ansprüche 3 bis 7, **dadurch gekennzeichnet, dass** der in Verfahrensschritt (c) verwendete Isomerisierungskatalysator der in Verfahrensschritt (a) verwendete Nickel(0)-Katalysators mit Chelatliganden ist.

9. Verfahren nach den Ansprüchen 1 bis 8, **dadurch gekennzeichnet, dass** man die Hydrocyanierung in Gegenwart von zusätzlichen monodentaten phosphorhaltigen Liganden, ausgewählt aus der Gruppe, bestehend aus Phosphinen, Phosphiten, Phosphiniten und Phosphoniten durchführt.

10. Verfahren nach Anspruch 9, **dadurch gekennzeichnet, dass** man als zusätzlichen monodentaten phosphorhaltigen Ligand ein Ligand der Formel II

    $$P(X^1R^1)(X^2R^2)(X^3R^3) \qquad (II),$$

    in der
    $X^1$,$X^2$,$X^3$ unabhängig voneinander Sauerstoff oder Einzelbindung und $R^1$,$R^2$,$R^3$ unabhängig voneinander gleiche oder unterschiedliche organische Reste bedeuten oder deren Gemische, einsetzt.

11. Verfahren nach den Ansprüchen 9 und 10, **dadurch gekennzeichnet, dass** man Verbindungen der Formel II a

$$(o\text{-Tolyl-O-})_w(m\text{-Tolyl-O-})_x(p\text{-Tolyl-O-})y(\text{Phenyl-O-})_zP \qquad \text{(IIa)}$$

einsetzt, wobei w, x, y und z eine natürliche Zahl bedeuten und folgende Bedingungen gelten:

$$w + x + y + z = 3 \text{ und } w, z \leq 2.$$

**12.** Verfahren nach den Ansprüchen 9 bis 11, **dadurch gekennzeichnet, dass** der zusätzliche monodentate phosphorhaltige Ligand des Nickel(0)-Komplexes und/oder der zusätzliche monodentate freie phosphorhaltige Ligand ausgewählt ist aus Tritolylphosphit sowie den Phosphiten der Formel II b

$$P(O\text{-}R^1)_x(O\text{-}R^2)_y(O\text{-}R^3)_z(O\text{-}R^4)_p \qquad \text{(II b),}$$

worin $R^1$, $R^2$ und $R^3$ unabhängig voneinander o-Isopropyl-phenyl, m-Tolyl und p-Tolyl, $R^4$ Phenyl ist; x 1 oder 2 ist, und y, z, p unabhängig voneinander 0, 1 oder 2 sind mit der Maßgabe, dass x + y + z + p = 3 ist, und deren Mischungen.

## Claims

**1.** A process for continuously hydrocyanating 1,3-butadiene in the presence of at least one catalyst, which comprises using, as catalysts, nickel(0) catalysts stabilized with phosphorus chelate ligands, 1,3-butadiene and hydrogen cyanide in a molar ratio of from 1.6:1 to 1.1:1 and the continuous hydrocyanation being additionally carried out in the presence of at least one Lewis acid.

**2.** The process according to claim 1, wherein the nickel(0) catalyst is saturated with phosphorus chelate ligands, the phosphorus chelate ligands being selected from the group consisting of bidentate phosphites, phosphines, phosphonites, phosphinites and phosphinite phosphites.

**3.** The process according to claim 1 or 2, **characterized by** the following process steps:

(a) continuously hydrocyanating 1,3-butadiene in the presence of at least one nickel(0) catalyst having chelate ligands and optionally in the presence of at least one Lewis acid, 1,3-butadiene and hydrogen cyanide being used in a molar ratio of from 1.6:1 to 1.1:1 to obtain a mixture 1 which comprises 3-pentenenitrile and 2-methyl-3-butenenitrile;
(c) continuously isomerizing 2-methyl-3-butenenitrile which is present in the mixture 1 over at least one dissolved or dispersed isomerization catalyst to give 3-pentenenitrile, resulting in a mixture 2.

**4.** The process according to claim 3, wherein the 3-pentenenitrile obtained in process step (c) is hydrocyanated in the presence of at least one nickel(0) catalyst having phosphorus ligands.

**5.** The process according to claim 3 or 4, wherein the isomerization in process step (c) is effected by heating the mixture 1 to from 80 to 125°C.

**6.** The process according to any of claims 3 to 5, wherein the continuous isomerization carried out in process step (c) is carried out in the presence of at least one Lewis acid.

**7.** The process according to any of claims 3 to 6, wherein, between process step (a) and process step (c), the following process step (b) is run through:

(b) distillatively removing 1,3-butadiene from the mixture 1.

**8.** The process according to any of claims 3 to 7, wherein the isomerization catalyst used in process step (c) is the nickel(0) catalyst having chelate ligands used in process step (a).

**9.** The process according to claims 1 to 8, wherein the hydrocyanation is carried out in the presence of additional monodentate phosphorus ligands selected from the group consisting of phosphines, phosphites, phosphinites and phosphonites.

**10.** The process according to claim 9, wherein the additional monodentate phosphorus ligand used is a ligand of the formula II

$$P(X^1R^1)(X^2R^2)(X^3R^3) \qquad (II)$$

in which
$X^1$, $X^2$, $X^3$ are each independently oxygen or a single bond and $R^1$, $R^2$, $R^3$ are each independently identical or different organic radicals, or mixtures thereof.

**11.** The process according to claims 9 and 10, wherein compounds of the formula IIa

$$(o\text{-tolyl-O-})_w(m\text{-tolyl-O-})_x(p\text{-tolyl-O-})y(\text{phenyl-O-})_2P \qquad (IIa)$$

are used, where w, x, y, z are each a natural number and the following conditions apply:

$$w + x + y + z = 3 \text{ and } w,\ z \geq 2.$$

**12.** The process according to claims 9 to 11, wherein the additional monodentate phosphorus ligand of the nickel(0) complex and/or the additional monodentate free phosphorus ligand is selected from tritolyl phosphite and the phosphites of the formula IIb

$$P(O\text{-}R^1)_x(O\text{-}R^2)_y(O\text{-}R^3)_2(O\text{-}R^4)_p \qquad (IIb)$$

where $R^1$, $R^2$ and $R^3$ are each independently o-isopropylphenyl, m-tolyl and p-tolyl, $R^4$ is phenyl, x is 1 or 2 and y, z, p are each independently 0, 1 or 2, with the proviso that x + y + z + p = 3, and mixtures thereof.

**Revendications**

**1.** Procédé d'hydrocyanation continue de 1,3-butadiène en présence d'au moins un catalyseur, **caractérisé en ce que** des catalyseurs de nickel (0), qui sont stabilisés avec des ligands chélateurs contenant du phosphore, sont utilisés en tant que catalyseurs, le 1,3-butadiène et le cyanure d'hydrogène sont utilisés en un rapport molaire de 1,6 à 1,1 sur 1, et l'hydrocyanation continue est en outre réalisée en présence d'au moins un acide de Lewis.

**2.** Procédé selon la revendication 1, **caractérisé en ce que** le catalyseur de nickel (0) est saturé avec des ligands chélateurs contenant du phosphore, les ligands chélateurs contenant du phosphore étant choisis dans le groupe constitué par les phosphites, phosphines, phosphonites, phosphinites et phosphinite-phosphites bidentates.

**3.** Procédé selon la revendication 1 ou 2, **caractérisé par** les étapes de procédé suivantes :

(a) l'hydrocyanation continue de 1,3-butadiène en présence d'au moins un catalyseur de nickel (0) comprenant des ligands chélateurs et éventuellement en présence d'au moins un acide de Lewis, le 1,3-butadiène et le cyanure d'hydrogène étant utilisés en un rapport molaire de 1,6 à 1,1 sur 1, et un mélange 1 étant obtenu, qui contient du 3-pentène-nitrile et du 2-méthyl-3-butène-nitrile ;
(c) l'isomérisation continue du 2-méthyl-3-butène-nitrile, qui est contenu dans le mélange 1, sur au moins un catalyseur d'isomérisation dissous ou dispersé, en 3-pentène-nitrile, un mélange 2 en résultant.

**4.** Procédé selon la revendication 3, **caractérisé en ce que** le 3-pentène-nitrile obtenu à l'étape de procédé (c) est hydrocyané en présence d'au moins un catalyseur de nickel (0) comprenant des ligands contenant du phosphore.

**5.** Procédé selon la revendication 3 ou 4, **caractérisé en ce que** l'isomérisation à l'étape de procédé (c) a lieu par chauffage du mélange 1 à 80 à 125 °C.

**6.** Procédé selon l'une quelconque des revendications 3 à 5, **caractérisé en ce que** l'isomérisation continue réalisée à l'étape de procédé (c) est réalisée en présence d'au moins un acide de Lewis.

7. Procédé selon l'une quelconque des revendications 3 à 6, **caractérisé en ce qu'**entre l'étape de procédé

(a) et l'étape de procédé (c), l'étape de procédé (b) suivante est réalisée :
(b) l'élimination par distillation de 1,3-butadiène du mélange 1.

8. Procédé selon l'une quelconque des revendications 3 à 7, **caractérisé en ce que** le catalyseur d'isomérisation utilisé à l'étape de procédé (c) est le catalyseur de nickel (0) comprenant des ligands chélateurs utilisé à l'étape de procédé (a).

9. Procédé selon les revendications 1 à 8, **caractérisé en ce que** l'hydrocyanation est réalisée en présence de ligands monodentates contenant du phosphore supplémentaires, choisis dans le groupe constitué par les phosphines, les phosphites, les phosphinites et les phosphonites.

10. Procédé selon la revendication 9, **caractérisé en ce qu'**en tant que ligand monodentate contenant du phosphore supplémentaire, un ligand de formule II

$$P(X^1R^1)(X^2R^2)(X^3R^3) \qquad (II)$$

est utilisé, dans laquelle
$X^1$, $X^2$, $X^3$ signifient indépendamment les uns des autres l'oxygène ou une liaison individuelle, et $R^1$, $R^2$, $R^3$ signifient indépendamment les uns des autres des radicaux organiques identiques ou différents, ou leurs mélanges.

11. Procédé selon les revendications 9 et 10, **caractérisé en ce que** des composés de formule IIa

$$(\text{o-tolyl-O-})_w(\text{m-tolyl-O-})_x(\text{p-tolyl-O-})_y(\text{phényl-O-})_zP \qquad (IIa)$$

sont utilisés, dans laquelle w, x, y, et z signifient un nombre naturel et ont les significations suivantes :

$$w + x + y + z = 3 \text{ et } w, z \leq 2.$$

12. Procédé selon les revendications 9 à 11, **caractérisé en ce que** le ligand monodentate contenant du phosphore supplémentaire du complexe de nickel (0) et/ou le ligand monodentate libre contenant du phosphore supplémentaire sont choisis parmi le tritolylphosphite, ainsi que les phosphites de formule IIb

$$P(O\text{-}R^1)_x(O\text{-}R^2)_y(O\text{-}R^3)_z(O\text{-}R^4)_p \qquad (IIb),$$

dans laquelle $R^1$, $R^2$ et $R^3$ signifient indépendamment les uns des autres o-isopropyl-phényle, m-tolyle et p-tolyle, $R^4$ signifie phényle ; x signifie 1 ou 2, et y, z, p signifient indépendamment les uns des autres 0, 1 ou 2, à condition que x + y + z + p = 3, et leurs mélanges.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9736855 A
- WO 9827054 A
- US 5723641 A
- US 5512696 A
- US 5821378 A
- US 5512695 A
- US 5981772 A
- US 6127567 A
- US 6020516 A
- US 5959135 A
- US 5847191 A
- US 5523453 A
- WO 0114392 A
- WO 9913983 A
- WO 9964155 A
- DE 10038037
- DE 10046025
- DE 10150285
- DE 10150286
- DE 10207165
- US 20030100442 A1
- DE 10350999
- DE 19953058 A
- DE 10351000
- DE 10351002
- DE 10351003
- US 6171996 B
- US 6380421 B
- US 3496217 A
- US 3496218 A
- US 4774353 A
- US 3773809 A
- US 4874884 A
- DE 102004004682 A


### In der Beschreibung aufgeführte Nicht-Patentliteratur


- **W. C. SEIDEL ; C. A. TOLEMAN.** Annals of the New York Academy of Science. *Catalytic Transition Metal Hydrides,* 1983, vol. 415, 201-221
- **KIRK-OTHMER.** Encyclopedia of Chemical Technology. John Wiley & Sons, 1996, vol. 20, 1040-1055
- **KIRK-OTHMER.** Encyclopedia of Chemical Technology. John-Wiley & Sons, 1996, vol. 20, 1040-1055